# EUROPÄISCHE PATENTSCHRIFT

(11) **EP 0 710 705 B1**
(45) Veröffentlichungstag und Bekanntmachung des Hinweises auf die Patenterteilung: **22.12.1999**
(21) Anmeldenummer: 95810665.0
(22) Anmeldetag: 27.10.1995
(51) Int. Cl.: C09B 57/00, C07D 487/04, C08K 5/34

(54) **Gelbe Diketopyrrolopyrrolpigmente**
Yellow diketopyrrolopyrrole pigments
Pigments jaunes de diketopyrrolopyrrole

(30) Priorität: 04.11.1994 CH 330594
(43) Veröffentlichungstag der Anmeldung: 08.05.1996
(73) Patentinhaber: Ciba Specialty Chemicals Holding Inc., 4057 Basel (CH)
(72) Erfinder: Lamatsch, Bernd, Dr., 1723 Marly (CH); Wallquist, Olof, Dr., D-1723 Marly (CH); Schlöder, Ingo, 1753 Matran (CH)

(56) Entgegenhaltungen:
- DE-A- 3 908 312
- US-A- 4 783 540
- US-A- 4 810 802

## Beschreibung

Die vorliegende Erfindung betrifft neue gelbe Diketopyrrolopyrrole und ihre Verwendung als Pigmente.

1,4-Diketopyrrolo[3,4-c]pyrrole sind seit einigen Jahren als rote Pigmente mit ausgezeichneten Pigmenteigenschaften bekannt, so z.B. aus US-Patent 4 415 685, wo Diketopyrrolopyrrole der Formel 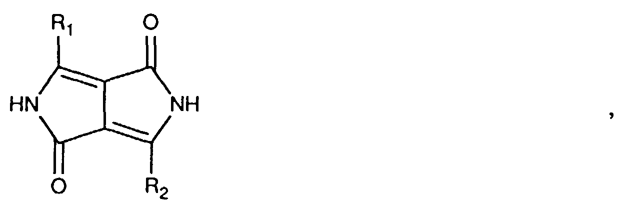 worin R₁ und R₂ isocyclische oder heterocyclische Reste, bevorzugt mono- bis tetracyclische, insbesondere mono- oder bicyclische Reste bedeuten, als rote Pigmente mit hoher Farbtonreinheit, hoher Farbstärke und guten Beständigkeiten, wie z.B. Licht-, Wetter-, Hitze- und Migrationsbeständigkeit, beschrieben sind. Die gute Eignung solcher Produkte als Pigmente mit oranger bis insbesondere roter Nuance wird auch in zahlreichen Folgepatenten, wie z.B. in US 4 579 949, US 4 720 305, US 4 810 802, US 4 783 540, DE-A-3 908 312 US 5 200 528, u.a.m., bestätigt.

Es ist nun gefunden worden, dass Diketopyrrolopyrrole der obenerwähnten Formel, worin die isocyclischen Reste unsubstituiertes oder substituiertes Phenanthren-9-yl bedeuten, ganz überraschend gelbe Pigmente mit ebenfalls sehr guten Pigmenteigenschaften sind.

Die vorliegende Erfindung betrifft demnach Diketopyrrolo[3,4-c]pyrrole der Formel 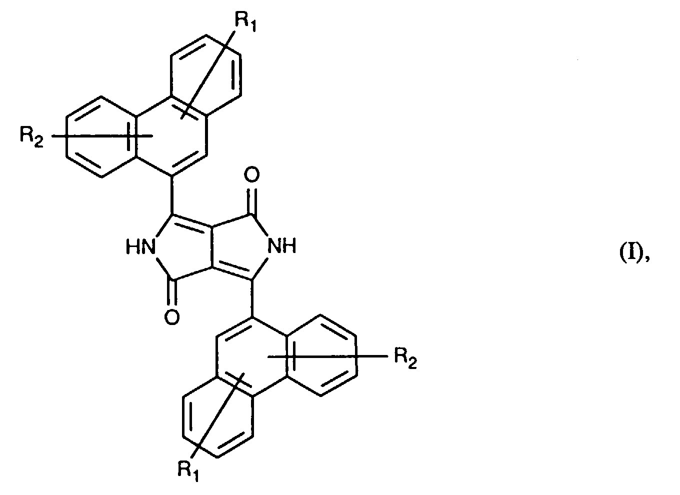 worin R₁ und R₂ unabhängig voneinander Wasserstoff, Halogen, C₁-C₆-Alkyl, Cyano oder eine Gruppe -OR₃, -COOR₃, -CON(R₃)(R₄), -COR₃ oder -N(R₃)(R₄) sind und R₃ und R₄ unabhängig voneinander Wasserstoff, C₁-C₆-Alkyl, unsubstituiertes oder durch Halogen oder C₁-C₆-Alkyl substituiertes Phenyl bedeuten.

Bedeuten etwaige Substituenten Halogen, so handelt es sich z.B. um Jod, Brom, Fluor oder insbesondere Chlor.

C₁-C₆-Alkyl steht z.B. für Methyl, Ethyl, n-Propyl, Isopropyl, n-Butyl, sec.-Butyl, tert.-Butyl, n-Pentyl, tert.-Amyl oder n-Hexyl.

Von besonderem Interesse sind die erfindungsgemässen 1,4-Diketopyrrolo[3,4-c]pyrrole der Formel I, worin R₁ Wasserstoff, Chlor, Brom, Methyl, Cyano, -N(R₃)₂ oder -OR₃ bedeutet, R₂ Wasserstoff ist und R₃ Wasserstoff oder Methyl bedeutet.

Bevorzugt sind die erfindungsgemässen Pigmente der Formel I worin R₁ und R₂ Wasserstoff bedeuten. Bevorzugte Positionen von Substituenten sind die 2-, die 3-, die 6- und die 7-Stellung am Phenanthryl-System.

Die erfindungsgemässen Diketopyrrolo[3,4-c]pyrrole der Formel I können in Analogie zu allgemein bekannten Verfahren hergestellt werden, z.B. durch Umsetzung eines Bemsteinsäurediesters mit einem Nitril der Formel 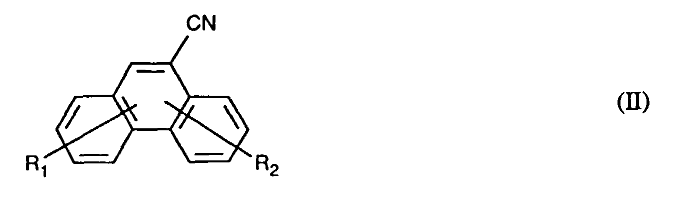 im erforderlichen Mengenverhältnis, wie beispielsweise aus dem US-Patent 4 579 949 bekannt.

Bei den Nitrilen der Formel II handelt es sich um bekannte Verbindungen. Sollten einige davon noch neu sein, so können sie in Analogie zu allgemein bekannten Methoden hergestellt werden.

Die erfindungsgemässen Diketopyrrolo[3,4-c]pyrrole können als Pigmente zum Färben von hochmolekularem organischem Material verwendet werden.

Hochmolekulare organische Materialien, die mit den erfindungsgemässen Diketopyrrolo-[3,4-c]pyrrolen pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkyldharze, Phenoplaste, Polycarbonate, Polyolefine, Polystyrol, Polyvinylchlorid, Polytetrafluoroethylen, Polyamide, Polyurethane, Polyester, Polyether-ketone, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Diketopyrrolo[3,4-c]pyrrole als Toner oder in Form von Präparaten einzusetzen. Bezogen auf das zu pigmentierende hochmolekulare organische Material kann man die erfindungsgemässen Diketopyrrolo[3,4-c]pyrrole in einer Menge von 0,01 bis 30 Gew.%, vorzugsweise von 0,1 bis 10 Gew.%, einsetzen.

Zum Pigmentieren von Lacken und Druckfarben werden die hochmolekularen organischen Materialien und die erfindungsgemässen Diketopyrrolo[3,4-c]pyrrole gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch gute allgemeine Eigenschaften, wie hohe Farbstärke, gute Dispergierbarkeit, gute Ueberlackier-, Migrations-, Hitze-, Licht- und Wetterbeständigkeit, sowie durch einen guten Glanz aus.

Die nachfolgenden Beispiele erläutern die Erfindung.

Beispiel 1: Zu 20 ml tert-Amylalkohol werden 48 ml einer 25 %-igen Lösung von Kalium-tert-amylat in Toluol zugegeben. Nach dem Aufheizen auf 87°C (Innentemperatur) werden 10,2 g 9-Cyanophenanthren zugefügt und während 25 Minuten 7,6 ml Bernsteinsäurediisopropylester zugetropft. Anschliessend lässt man bei dieser Temperatur 4 Stunden rühren. Die erhaltene Suspension wird unter kräftigem Rühren mit der Zahnscheibe in eine Mischung von 200 ml Wasser, 200 ml Methanol und 3 ml Essigsäure eingetragen und noch 10 Minuten nachgerührt. Nach Filtration, Waschen mit Methanol und Wasser und Trocknen bei 60°C im Vakuum erhält man 2,3 g (19 % d. Th.) des gelben Pigments der Formel

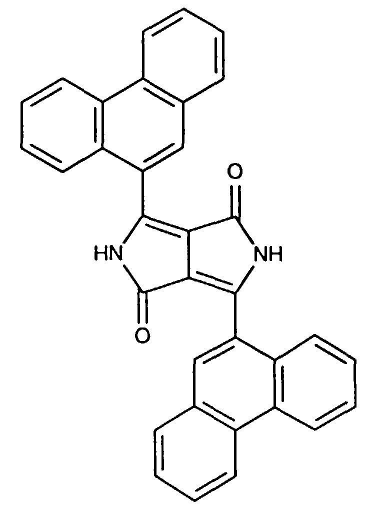

Beispiel 2: Eine Mischung von 1,0 g des nach Beispiel 1 erhaltenen Pigmentes, 1,0 g Antioxidans (IRGANOX® 1010, CIBA-GEIGY AG) und 1000 g Polyethylen-HD Granulat (®VESTOLEN 60-16, HUELS) wird während 15 Minuten in einer Glasflasche auf einer Rollbank vorgemischt. Danach wird die Mischung in zwei Passagen auf einem Einwellenextruder extrudiert, das so erhaltene Granulat wird auf der Spritzgussmaschine (Allround Aarburg 200) bei 220°C zu Platten verspritzt und 5 Minuten bei 180°C nachgepresst. Die Pressplatten weisen farbstarke gelbe Nuancen mit guten Beständigkeiten auf.

Beispiel 3: 0,6 g des gemäss Beispiel 1 erhaltenen Pigmentes werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte gelbe PVC-Folie ist farbstark und lichtbeständig.

Beispiel 4: 2 g des gemäss Beispiel 1 erhaltenen Produktes und 48 g Einbrennlack bestehend aus

| | |
|---|---|
| 56 g | Alkydharz ALKYDAL® F310 (Bayer AG; 60 %ig in Xylol) |
| 13 g | Melaminharz CYMEL® 327 (Cyanamid; 90 %ig in Butanol) |
| 25 g | Xylol |
| 25 g | Butanol |
| 2,5 g | 1-Methoxy-2-Propanol und |
| 1 g | Silikonöl (1 %ig in Xylol) |

werden nach üblichen Methoden vermischt. Die Applikation des erhaltenen Farblackes erfolgt durch Ablauf auf Glasplatte. Vor dem Einbrennen im Umluftrockenschrank (30 Minuten bei 120°C) lässt man ca. 30 Minuten bei 25° Neigung abluften.

Man erhält eine farbstarke gelbe Lackierung mit guten Allgemeinechtheiten.

## Patentansprüche

1. Diketopyrrolo[3,4-c]pyrrole der Formel 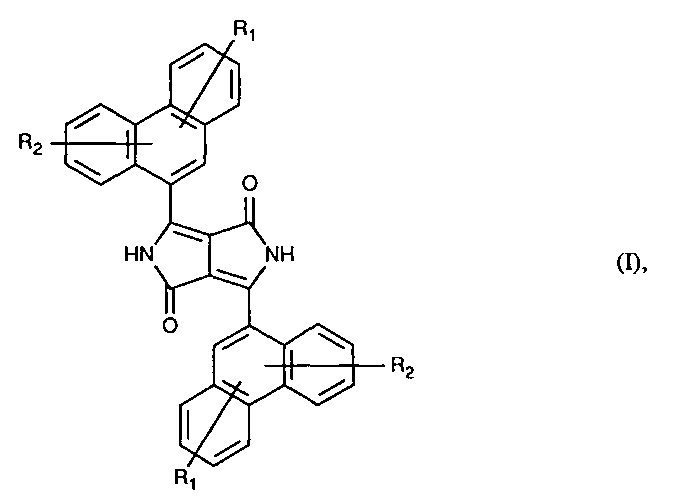 worin R₁ und R₂ unabhängig voneinander Wasserstoff, Halogen, C₁-C₆-Alkyl, Cyano oder eine Gruppe -OR₃, -COOR₃, -CON(R₃)(R₄), -COR₃ oder -N(R₃)(R₄) sind und R₃ und R₄ unabhängig voneinander Wasserstoff, C₁-C₆-Alkyl, unsubstituiertes oder durch Halogen oder C₁-C₆-Alkyl substituiertes Phenyl bedeuten.

2. Diketopyrrolo[3,4-c]pyrrole der Formel I gemäss Anspruch 1, worin worin R₁ Wasserstoff, Chlor, Brom, Methyl, Cyano, -N(R₃)₂ oder -OR₃ bedeutet, R₂ Wasserstoff ist und R₃ Wasserstoff oder Methyl bedeutet.

3. Diketopyrrolo[3,4-c]pyrrole der Formel I gemäss Anspruch 1, worin R₁ und R₂ Wasserstoff bedeuten.

4. Mit einem Diketopyrrolo[3,4-c]pyrrol gemäss Anspruch 1 pigmentiertes hochmolekulares organisches Material.

## Claims

1. A diketopyrrolo[3,4-c]pyrrole of formula 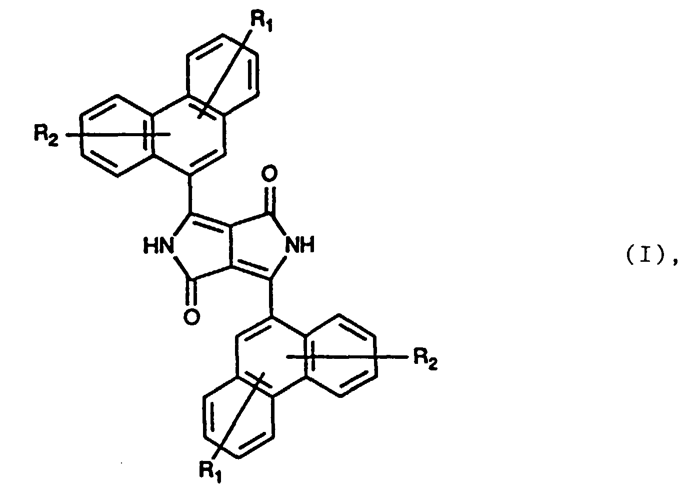 wherein R₁ and R₂ are each independently of one another hydrogen, halogen, C₁-C₆alkyl, cyano or a -OR₃, -COOR₃, -CON(R₃) (R₄), -COR₃ or -N(R₃) (R₄) group, and R₃ and R₄ are each independently of one another hydrogen, C₁-C₆alkyl, or phenyl which is unsubstituted or substituted by halogen or C₁-C₆alkyl.

2. A diketopyrrolo[3,4-c]pyrrole of formula I according to claim 1, wherein R₁ is hydrogen, chloro, bromo, methyl, cyano, -N(R₃)₂ or -OR₃, R₂ is hydrogen, and R₃ is hydrogen or methyl.

3. A diketopyrrolo[3,4-c]pyrrole of formula I according to claim 1, wherein R₁ and R₂ are hydrogen.

4. A high molecular mass organic material pigmented with a diketopyrrolo[3,4-c]pyrrole according to claim 1.

## Revendications

1. Dicétopyrrolo[3,4-c]pyrroles de formule 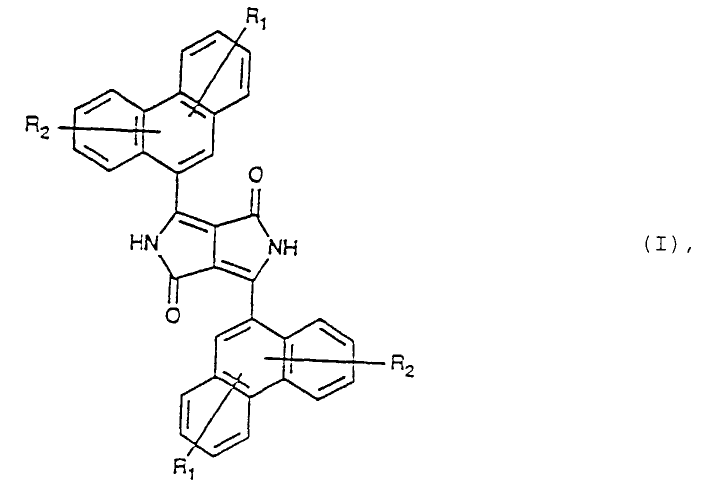 où R₁ et R₂ représentent, indépendamment l'un de l'autre, hydrogène, halogène, alkyle en C₁-C₆, cyano ou un groupe -OR₃, -COOR₃, -CON(R₃)(R₄), -COR₃ ou -N(R₃)(R₄), et où R₃ et R₄ représentent, indépendamment l'un de l'autre, hydrogène, alkyle en C₁-C₆ ou phényle non substitué ou substitué par halogène ou alkyle en C₁-C₆.

2. Dicétopyrrolo[3,4-c)pyrroles de formule I selon la revendication 1, où R₁ représente hydrogène, chlore, brome, méthyle, cyano, -N(R₃)₂ ou -OR₃, R₂ est hydrogène et R₃ est hydrogène ou méthyle.

3. Dicétopyrrolo[3,4-c)pyrroles de formule I selon la revendication 1, où R₁ et R2 représentent hydrogène.

4. Matériau organique à haut poids moléculaire pigmenté par un dicétopyrrolo[3,4-c)pyrrole selon la revendication 1.
